# EUROPÄISCHE PATENTSCHRIFT

(11) **EP 1 079 751 B1**
(45) Veröffentlichungstag und Bekanntmachung des Hinweises auf die Patenterteilung: **28.02.2007**
(21) Anmeldenummer: 98919022.8
(22) Anmeldetag: 19.05.1998
(51) Int. Cl.: A61B 17/64

(54) **BACKE FÜR MONOLATERALES EXTERNES FIXATIONSSYSTEM FÜR TRAUMATOLOGIE UND ORTHOPÄDIE**
CHEEK FOR A ONE-SIDED EXTERNAL FIXATION SYSTEM FOR TRAUMATOLOGY AND ORTHOPEDICS
MACHOIRE DESTINEE A UN SYSTEME DE FIXATION EXTERNE MONOLATERAL UTILISE EN TRAUMATOLOGIE ET EN ORTHOPEDIE

(43) Veröffentlichungstag der Anmeldung: 07.03.2001
(73) Patentinhaber: Synthes GmbH, 4436 Oberdorf (CH)
(72) Erfinder: MARTINELLI, Orlando, CH-3018 Bern (CH); INAUEN, Beat, CH-4434 Hölstein (CH); FLÜHLER, Erwin, CH-4123 Allschwil (CH); CLAES, Lutz, D-89233 Neu-Ulm (DE); GERNGROSS, Heinz, D-89075 Ulm (DE); RÜBSAAMEN, Götz, D-83278 Traunstein (DE)
(74) Vertreter: Lusuardi, Werther
(86) Internationale Anmeldenummer: PCT/CH1998/000207
(87) Internationale Veröffentlichungsnummer: WO 1999/059490

(56) Entgegenhaltungen:
- EP-A- 0 420 430
- DE-U- 8 914 594
- US-A- 5 207 676

## Beschreibung

Die Erfindung bezieht sich auf eine Vorrichtung zum äusseren Festlegen von Teilen von gebrochenen Knochen gemäss dem Oberbegriff des Patentanspruchs 1.

Aus dem Stand der Technik ist aus der EP 0 011 258 A1 ORTHOFIX eine Vorrichtung zum äusseren Festlegen der Teile eines gebrochenen Knochens bekannt. Diese bekannte Erfindung umfasst einen langgestreckten Mittelkörper, der aus zwei parallel zu seiner Längsachse gegeneinander verschiebbaren Teilen besteht, wobei jeder Teil des Mittelkörpers eine Klemmeinrichtung für in einen Knochenteil einsetzbare Nägel oder Knochenschrauben trägt, und eine an den beiden Teilen des Mittelkörpers angreifende Druck-und Zugeinrichtung. Die Klemmeinrichtungen sind mittels Universalgelenken respektive Kugelgelenken an den äusseren Enden der verschiebbaren Teile des Mittelkörpers gelagert, was eine Schwenkbarkeit der Klemmeinrichtungen und der in die Knochenteile einsetzbaren Nägel oder Schrauben mit drei Freiheitsgraden ermöglicht. Der Nachteil dieser bekannten Erfindung liegt darin, dass die Klemmeinrichtungen nur mittels Reibschluss fixiert werden, was die Rutschfestigkeit der einmal fixierten Vorrichtung erheblich verringert.

Eine weitere aus dem Stand der Technik bekannte Vorrichtung zur externen Fixierung von Knochen mittels Stiften ist in der DE-U- 89 14 594.1 offenbart.

Eine weitere aus dem Stand der Technik bekannte Vorrichtung zur externen Fixierung von Knochen mittels Stiften ist in der US 5,160,335 WAGENKNECHT offenbart. Diese bekannte Erfindung umfasst einen Fixationsstab, der auch teleskopierbar sein kann, mit Klemmelementen zum Festhalten der Knochenstifte, Verbindungsteile mit gekrümmten Auflageflächen für die Klemmelemente und Klammern zur Befestigung der Klemmelemente mit den Verbindungsteilen auf dem Fixationsstab. Die Verbindungsteile sind so gestaltet, dass die Klemmelemente gegenüber den Klammern mit drei Freiheitsgraden schwenkbar sind. Wiederum liegt ein Nachteil dieser Erfindung darin, dass die Schwenkgelenke nur durch Reibschluss gegeneinander fixierbar sind und zudem drei Schrauben angezogen werden müssen, um die Klemmelemente zu blockieren.

Hier will die Erfindung Abhilfe schaffen. Der Erfindung liegt die Aufgabe zugrunde, einerseits die Fixierung der Schwenkgelenke mittels Formschluss zu ermöglichen und andererseits die lösbare Blockierung von zwei oder mehr rotativen Freiheitsgraden mit einer einzigen Feststellschraube zu ermöglichen.

Die Erfindung löst die gestellte Aufgabe mit einer Vorrichtung zum äusseren Festlegen von Teilen von gebrochenen Knochen, welche die Merkmale des Anspruchs 1 aufweist.

Weitere vorteilhafte Ausgestaltungen der Erfindung sind in den abhängigen Ansprüchen gekennzeichnet.

In einer bevorzugten Ausführungsform der erfindungsgemässen Vorrichtung umfasst diese eine Backe und drei entlang einer Achse angeordnete Schwenkelemente, wobei zwischen der Backe und einer Spannfläche am oberen der Schwenkelemente Knochenschrauben oder Stifte eingespannt werden können und die gesamte Vorrichtung auf einem longitudinalen Verbindungselement fixierbar ist. Das obere Schwenkelement ist gegenüber dem mittleren Schwenkelement um eine Drehachse, welche quer zur Achse verläuft, drehbar und gegen eine als Drehgelenk geformte Fläche des mittleren Schwenkelementes klemmbar und in einer gewünschten Position fixierbar. Das untere Schwenkelement ist gegenüber dem mittleren Schwenkelement um die Achse drehbar und gegen eine zweite Stirnfläche des mittleren Schwenkelementes klemmbar und in einer gewünschten Position fixierbar. Eine quer zur Achse verlaufende durchgehende Bohrung im unteren Schwenkelement ermöglicht, dass das untere Schwenkelement und damit die gesamte erfindungsgemässe Vorrichtung verschiebbar und um die Achse der Bohrung drehbar auf ein Verbindungselement aufgesetzt und in einer beliebigen Position fixiert werden kann. Die Fixation des unteren Schwenkelementes auf dem Verbindungselement und die Fixation der drei Schwenkelemente gegeneinander erfolgt mittels einer einzigen Feststellschraube. Zur formschlüssigen Verbindung zwischen jeweils zwei Schwenkelementen sind diese an den sich jeweils gegenseitig berührenden Kontaktflächen mit Verzahnungen versehen.

Eine andere Ausführungsform der erfindungsgemässen Vorrichtung unterscheidet sich von der oben beschriebenen Ausführungsform nur dadurch, dass die Drehachse zwischen dem oberen und dem mittleren Schwenkelement senkrecht zur Achse, entlang welcher die Schwenkelemente angeordnet sind, verläuft.

Wieder eine andere Ausführungsform der erfindungsgemässen Vorrichtung unterscheidet sich von den vorangehend beschriebenen Ausführungsformen nur darin, dass die Bohrung, welche das Aufsetzen des unteren Schwenkelementes auf das Verbindungselement ermöglicht, senkrecht zur Achse, entlang welcher die Schwenkelemente angeordnet sind, verläuft.

Eine weitere Ausführungsform der erfindungsgemässen Vorrichtung unterscheidet sich von den oben beschriebenen Ausführungsformen nur dadurch, dass die mit dem oberen Schwenkelement in Berührung stehende Stirnfläche des mittleren Schwenkelementes und die mit dem unteren Schwenkelement in Berührung stehende Stirnfläche des mittleren Schwenkelementes parallel sind.

Wiederum eine weitere Ausführungsform der erfindungsgemässen Vorrichtung unterscheidet sich von der vorangehend beschriebenen Ausführungsform nur darin, dass diese einzige Feststellschraube mit dem Schraubenkopf auf einer senkrecht zur Achse stehenden Fläche am unteren Schwenkelement aufliegt und in ein zum Gewinde der Feststellschraube korrespondierendes Gewinde in einem im oberen Schwenkelement drehbar gelagerten kreissegmentförmigen Einlageteil schraubbar ist.

Wiederum eine andere Ausführungsform der erfindungsgemässen Vorrichtung unterscheidet sich von der vorhergehend beschriebenen Ausführungsform nur darin, dass die einzige Feststellschraube mit dem Schraubenkopf auf einer Auflagefläche des im oberen Schwenkelement drehbar gelagerten kreissegmentförmigen Einlageteils aufliegt und in ein zum Gewinde der Feststellschraube korrespondierendes Gewinde im unteren Schwenkelement schraubbar ist.

Wiederum eine andere Ausführungsform der erfindungsgemässen Vorrichtung unterscheidet sich von den vorangehend beschriebenen Ausführungsformen nur darin, dass das untere Schwenkelement einseitig mit einem bis zur Bohrung durchgehenden Schlitz versehen ist und eine Feststellschraube ermöglicht, dass der Schlitz und damit auch die Bohrung zusammengepresst wird. Auf diese Weise lässt sich das untere Schwenkelement in einer gewünschten Position auf dem Verbindungselement festklemmen.

Wiederum eine weitere Ausführungsform der erfindungsgemässen Vorrichtung unterscheidet sich von den vorangehend beschriebenen Ausführungsformen nur dadurch, dass die am unteren Schwenkelement an der Kontaktfläche mit dem mittleren Schwenkelement und die am mittleren Schwenkelement an der Kontaktfläche mit dem unteren Schwenkelement angebrachten Verzahnungen kreisringförmig und konzentrisch zur Achse ausgebildet sind.

Wiederum eine andere Ausführungsform der erfindungsgemässen Vorrichtung unterscheidet sich von der oben beschriebenen Ausführungsform nur dadurch, dass die am mittleren Schwenkelement an der Kontaktfläche mit dem oberen Schwenkelement angebrachte Verzahnung als kreissegmentförmige Vertiefung mit einer Drehachse senkrecht zur Achse ausgebildet ist und die am oberen Schwenkelement an der Kontaktfläche mit dem mittleren Schwenkelement angebrachte Verzahnung als halbkreisförmige Ausbuchtung mit einer Drehachse senkrecht zur Achse ausgebildet ist.

Wiederum eine weitere Ausführungsform der erfindungsgemässen Vorrichtung unterscheidet sich von den vorangehend beschriebenen Ausführungsformen nur darin, dass die Knochenschrauben zwischen der Backe und dem oberen Schwenkelement durch Spannelemente, vorzugsweise Spannschrauben unabhängig von der Fixierung der Schwenkelemente fixierbar sind.

Wiederum eine andere Ausführungsform der erfindungsgemässen Vorrichtung unterscheidet sich von den vorangehend beschriebenen Ausführungsformen nur dadurch, dass die am oberen Schwenkelement angebrachte Spannfläche auf ihrer Breite mit parallelen Vertiefungen zur teilweisen Aufnahme der Knochenschrauben und die Backe auf der der Spannfläche am oberen Schwenkelement zugewandten Seite auf ihrer Breite mit parallelen Vertiefungen zur teilweisen Aufnahme der Knochenschrauben versehen ist.

Wiederum eine weitere Ausführungsform der erfindungsgemässen Vorrichtung unterscheidet sich von den vorangehend beschriebenen Ausführungsformen nur darin, dass die Feststellschraube auf beiden Stirnflächen mit je einem zur Aufnahme eines Werkzeuges zum Lösen und Anziehen der Feststellschraube dienenden Mittel, welches beispielsweise als Innensechskant, Schraubenschlitz oder Kreuzschlitz ausgebildet sein kann, versehen ist.

Die durch die Erfindung erreichten Vorteile sind im wesentlichen darin zu sehen, dass dank der erfindungsgemässen Vorrichtung die Backen und die Schwenkelemente, welche die Knochenschrauben aufnehmen und mit dem longitudinalen Verbindungselement verbinden, mit formschlüssig fixierbaren Schwenkgelenken versehen sind. Die in die Vorrichtung mittels Feststellelementen integrierten Schwenkgelenke sind zudem so gestaltet, dass zwei oder alle drei rotativen Freiheitsgrade mit einer einzigen Feststellschraube blockiert werden können.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Aufriss einer Ausführungsform der erfindungsgemässen Vorrichtung mit einem verbindungselement;
Fig. 2 einen Schnitt durch eine Ausführungsform einer nicht-erfindungsgemässen Vorrichtung;
Fig. 3 einen Schnitt durch eine Ausführungsform der erfindungsgemässen Vorrichtung; und
Fig. 4 einen Schnitt durch eine weitere Ausführungsform der erfindungsgemässen Vorrichtung.

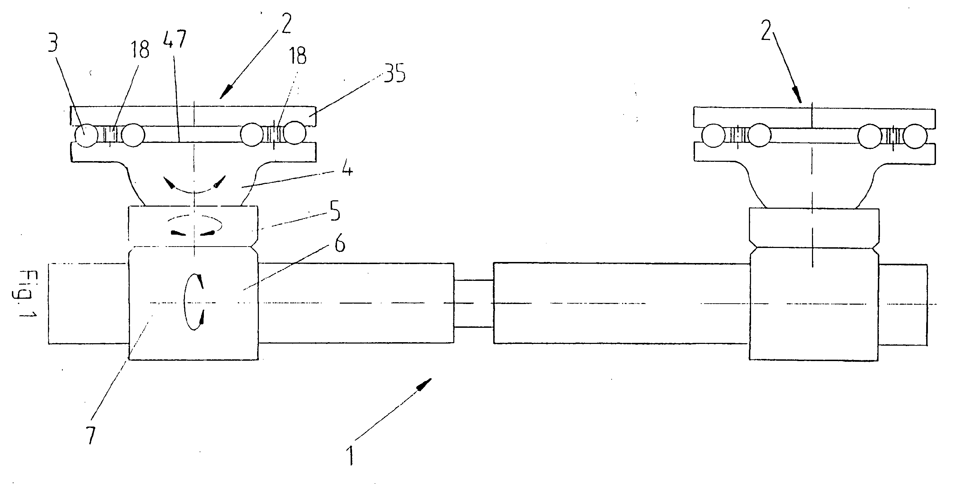

Fig 1. zeigt eine Ausführungsform der erfindungsgemässen Vorrichtung 2, welche auf einem Verbindungselement 1 bewegbar und fixierbar ist. Die Vorrichtung 2 ist mit drei Schwenkelementen 4;5;6 versehen, wodurch eine Bewegung der in der Vorrichtung 2 eingespannten Knochenschrauben 3 mit drei rotativen Freiheitsgraden gegenüber dem Verbindungselement 1 ermöglicht wird. Das Schwenkelement 4 ist so gestaltet, dass vier Knochenschrauben 3 eingespannt werden können. Die Knochenschrauben 3 werden zwischen der Spannfläche 47 auf dem Schwenkelement 4 und der Backe 35 eingespannt, wobei die Backe 35 mittels Spannschrauben 18 gegen das Schwenkelement 4 gepresst wird.

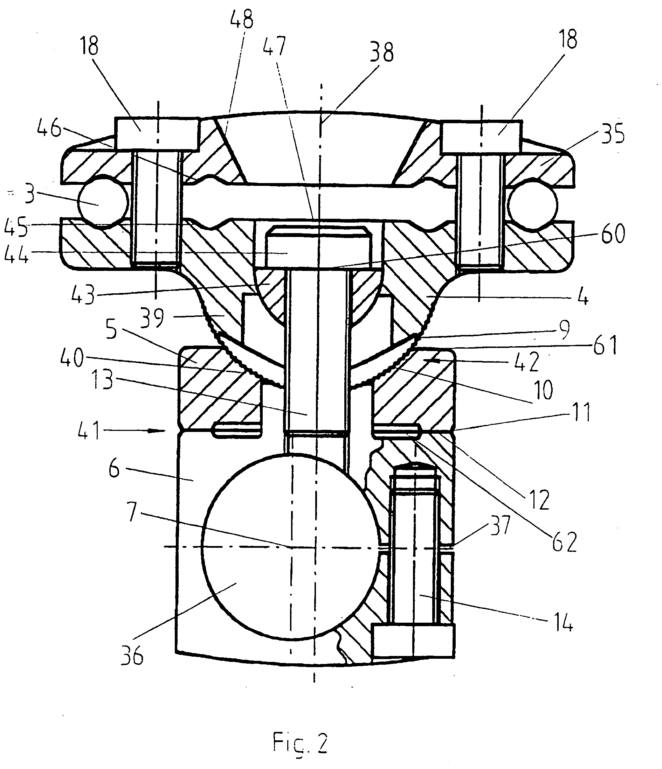

In Fig. 2 ist eine Ausführungsform einer nichts-erfindungsgemässen Vorrichtung dargestellt. Die Schwenkelemente 4;5;6 ermöglichen eine Bewegbarkeit der Vorrichtung 2 mit drei rotativen Freiheitsgraden. Das Schwenkelement 6 besteht aus einem Grundkörper und ist mit einer durchgehenden Bohrung 36 versehen, welche im Durchmesser so gestaltet ist, dass das Schwenkelement 6 auf dem Verbindungselement 1 verschiebbar und drehbar ist. An einer gewünschten Position auf dem Verbindungselement 1 kann das einseitig bis zur Bohrung 36 durchgehend mit einem Schlitz 37 versehene Schwenkelement 6 mittels der Feststellschraube 14 auf dem Verbindungselement 1 festgeklemmt werden. Durch diese lösbare Klemmverbindung ist eine Rotation der Vorrichtung 2 um die Zentralachse 7 der im Schwenkelement 6 angebrachten Bohrung 36 möglich. Die Schwenkelemente 4;5;6 der Vorrichtung 2 sind senkrecht zur Zentralachse 7 so angeordnet, dass jeweils ein Schwenkelement 4;5;6 über eine Kontaktfläche an das Nächste anstösst. Das Schwenkelement 5 ist gegenüber dem Schwenkelement 6 um eine Achse 38 rotierbar, was einen weiteren rotativen Freiheitsgrad der Vorrichtung 2 ermöglicht. Die Kontaktflächen zwischen den Schwenkelementen 5 und 6 sind mit formschlüssig ineinander eingreifenden Verzahnungen 11;12 versehen. Die Drehachse dieser Schwenkverbindung zwischen den Schwenkelementen 5 und 6 fällt mit der Achse 38 des Schwenkelementes 5 zusammen. Die Kontaktflächen zwischen den Schwenkelementen 4 und 5 sind kreissegmentförmig gestaltet, so dass am Schwenkelement 4 eine halbkreisförmige Ausbuchtung 39 angebracht ist, diese in eine kreissegmentförmige Vertiefung 40 am Schwenkelement 5 eingreift und eine rotative Bewegung der Standardbacke mit einem dritten Freiheitsgrad ermöglicht. Die Kontaktflächen zwischen den Schwenkelementen 4 und 5 sind ebenfalls mit formschlüssig ineinander eingreifenden Verzahnungen 9;10 ausgestattet. Fixiert werden die beiden Klemmverbindungen 41;42 mittels einem einzigen als Feststellschraube ausgebildeten Feststellelement 13, dessen Kopf 44 im Schwenkelement 4 mittels eines kreissegmentförmigen Einlageteils 43 gelagert ist und dessen Gewindeschaft in das Schwenkelement 6 eingeschraubt wird. Das Schwenkelement 4 ist auf der der Ausbuchtung 39 relativ zur Achse 38 gegenüberliegenden Spannfläche 47 auf seiner Breite mit parallelen Vertiefungen 45 zur teilweisen Aufnahme der Knochenschrauben 3 versehen. Die Knochenschrauben 3 werden zwischen dem Schwenkelement 4 und einer Backe 35, welche ebenfalls mit parallelen Vertiefungen 46 versehen ist, eingelegt und mittels Spannschrauben 18, deren Köpfe auf der Backe 35 aufliegen und die in das Schwenkelement 4 eingeschraubt werden, festgeklemmt. In der Backe 35 befindet sich eine Öffnung 48, welche ein Festziehen oder Lösen des Feststellelementes 13 zulässt auch wenn die Backe 35 auf dem Schwenkelement 4 festgeschraubt ist und Knochenschrauben 3 zwischen Schwenkelement 4 und Backe 35 eingespannt sind. Dadurch wird eine Bewegung der- fest eingespannten Knochenschrauben 3 gegenüber dem Verbindungselement 1 mit zwei rotativen Freiheitsgraden durch das Lösen des einen Feststellelementes 13 möglich.

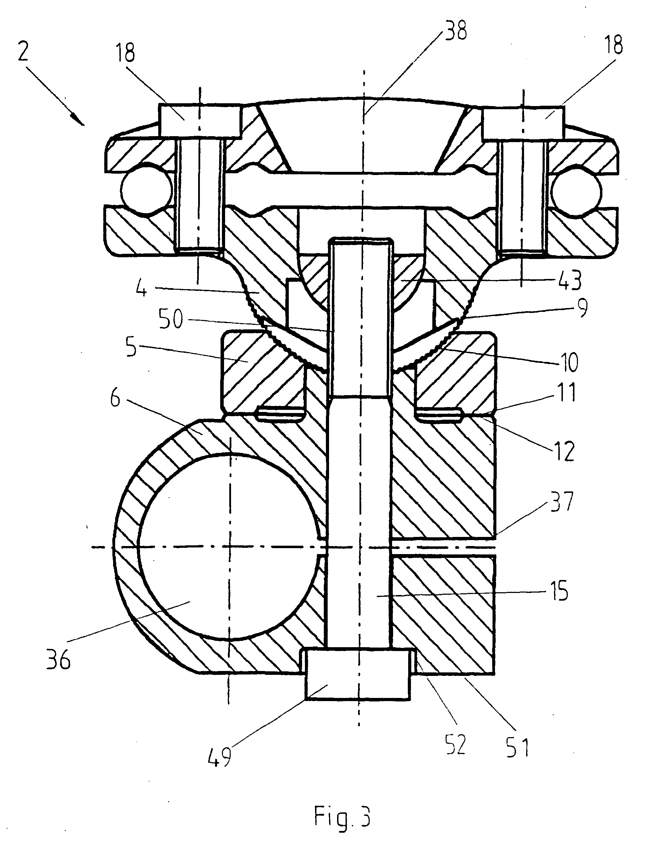

Die in Fig. 3 dargestellte Ausführungsform der erfindungsgemässen Vorrichtung unterscheidet sich von der in Fig. 2 gezeigten Ausführungsform nur dadurch, dass zwischen dem kreissegmentförmigen Einlageteil 43 im Schwenkelement 4 und dem Schwenkelement 6 nur ein einziges als Feststellschraube ausgebildetes Feststellelement 15 angebracht ist. Diese Feststellschraube 15 verläuft entlang der Achse 38. Der Schraubenkopf 49 steht am unteren Ende 51 in einer Ansenkung 52 auf. Das Gewinde 50 der Feststellschraube 15 wird in das kreissegmentförmige Einlageteil 43 eingeschraubt. Die Feststellschraube 15 fixiert alle drei Schwenkelemente 4;5;6. Beim Anziehen der Feststellschraube 15 wird der Schlitz 37 im Schwenkelement 6 zusammengepresst, wodurch das Schwenkelement 6 auf dem Verbindungselement 1 fixiert wird. Gleichzeitig wird beim Festziehen der Feststellschraube 15 sowohl die Schwenkverbindung 41 zwischen den Schwenkelementen 6 und 5 mit den formschlüssig ineinander eingreifenden Verzahnungen 11 und 12 als auch die Schwenkverbindung 42 zwischen den Schwenkelementen 5 und 4 mit den ebenfalls formschlüssig ineinander eingreifenden Verzahnungen 9 und 10 blockiert.

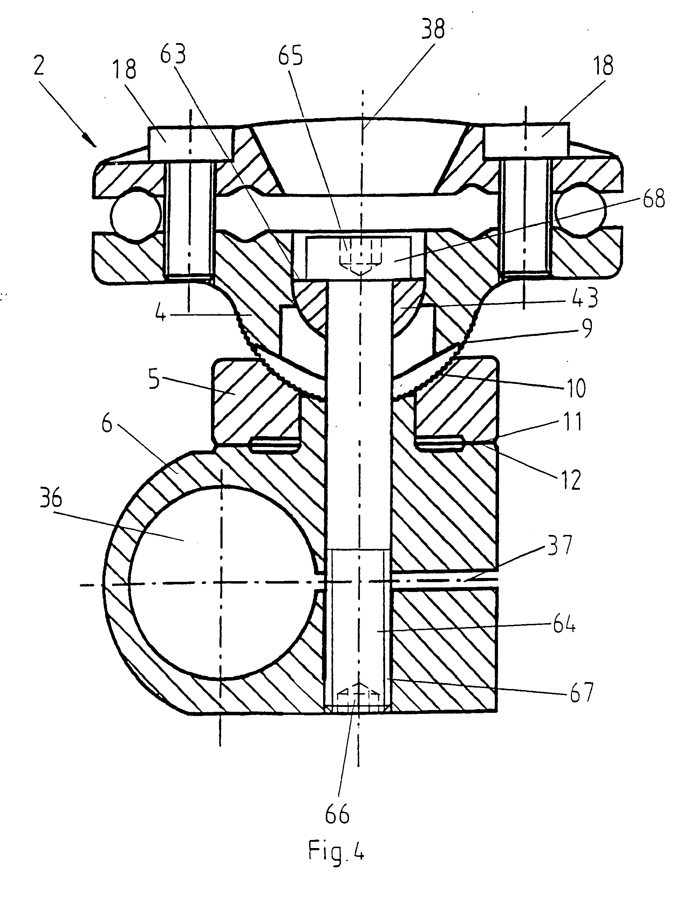

Die in Fig. 4 dargestellte Ausführungsform der erfindungsgemässen Vorrichtung unterscheidet sich von der in Fig. 3 gezeigten Ausführungsform nur darin, dass das als Feststellschraube ausgebildete Feststellelement 64 vom Schwenkelement 4 her in das Gewinde 67 im Schwenkelement 6 eingeschraubt wird. Der Schraubenkopf 68 der Feststellschraube 64 liegt auf dem im Schwenkelement 4 drehbar gelagerten kreissegmentförmigen Einlageteils 43 auf. Die Feststellschraube 64 ist auf beiden Stirnflächen mit je einem zur Aufnahme eines Werkzeuges zum Lösen und Anziehen der Feststellschraube dienenden Mittel (65;66), welches hier als Innensechskant ausgebildet ist, versehen und kann daher vom Schwenkelement 4 her wie auch vom Schwenkelement 6 her angezogen oder gelöst werden.

## Patentansprüche

1. Vorrichtung (2) zum äusseren Festlegen von Teilen von gebrochenen Knochen, welche auf einem longitudinalen Verbindungselement (1) fixierbar ist und eine Backe (35), ein oberes Schwenkelement (4), ein mittleres Schwenkelement (5) und ein unteres Schwenkelement (6) umfasst und die Schwenkelemente (4;5;6) entlang mindestens einer Achse (38) so angeordnet sind, dass sich je zwei Schwenkelemente (4;5;6) berühren, wobei
A) zwischen der Backe (35) und einer am oberen Schwenkelement (4) angeordneten Spannfläche (47) mindestens zwei Knochenschrauben (3) oder Stifte festklemmbar sind;
B) das obere Schwenkelement (4) gegenüber dem mittleren Schwenkelement (5) um eine Drehachse (60), welche quer zur Achse (38) verläuft, drehbar ist und gegen die erste quer zur Achse (38) stehende Stirnfläche (61) des mittleren Schwenkelementes (5) klemmbar ist;
C) das untere Schwenkelement (6) gegenüber dem mittleren Schwenkelement (5) um die Achse (38) drehbar ist und gegen dessen zweite Stirnfläche (62) klemmbar ist;
D) das untere Schwenkelement (6) mit einer quer zur Achse (38) verlaufenden durchgehenden Bohrung (36) versehen ist, welche gestattet, dass das untere Schwenkelement (6) auf dem Verbindungselement (1) verschiebbar und um die Zentralachse (7) der Bohrung (36) gegenüber dem Verbindungselement (1) rotierbar ist; und
E) das untere Schwenkelement (6) auf dem Verbindungselement (1) fixierbar ist;
**dadurch gekennzeichnet, dass**
F) mindestens zwei der Schwenkelemente (4;5;6) an den sich gegenseitig berührenden Kontaktflächen mit Verzahnungen (9;10;11;12) zur formschlüssigen Verbindung von jeweils zwei miteinander in Kontakt stehenden Schwenkelementen (4;5;6) versehen sind und alle drei Schwenkelemente (4;5;6) gegeneinander und die Vorrichtung (2) auf dem Verbindungselement (1) mit einem einzigen Feststellelement (15,64) fixierbar sind.

2. Vorrichtung nach Anspruch 1, **dadurch gekennzeichnet, dass** die Drehachse (60) senkrecht zur Achse (38) verläuft.

3. Vorrichtung nach Anspruch 1 oder 2, **dadurch gekennzeichnet, dass** die Bohrung (36) im unteren Schwenkelement (6) senkrecht zur Achse (38) verläuft.

4. Vorrichtung nach einem der Ansprüche 1 bis 3, **dadurch gekennzeichnet, dass** die Stirnflächen (61;62) des mittleren Schwenkelementes (5) parallel sind.

5. Vorrichtung nach einem der Ansprüche 1 bis 4, **dadurch gekennzeichnet, dass** das Feststellelement (15;64) eine Feststellschraube ist

6. Vorrichtung nach Anspruch 5, **dadurch gekennzeichnet, dass** das einzige Feststellelement (15) mit dem Schraubenkopf (49) auf einer senkrecht zur Achse (38) stehenden Fläche (52) am unteren Schwenkelement (6) aufliegt und in ein zum Gewinde (50) der Feststellschraube korrespondierendes Gewinde in einem im oberen Schwenkelement (4) drehbar gelagerten kreissegmentförmigen Einlageteil (43) schraubbar ist.

7. Vorrichtung nach Anspruch 5, **dadurch gekennzeichnet, dass** das einzige Feststellelement (64) mit dem Schraubenkopf (68) auf einer Auflagefläche (63) eines im oberen Schwenkelement (4) drehbar gelagerten kreissegmentförmigen Einlageteils (43) aufliegt und in ein zum Gewinde (67) der Feststellschraube korrespondierendes Gewinde im unteren Schwenkelement (6) schraubbar ist.

8. Vorrichtung nach einem der Ansprüche 1 bis 5, **dadurch gekennzeichnet, dass** das Schwenkelement (6) einseitig mit einem bis zur Bohrung (36) durchgehenden Schlitz (37) versehen ist und ein Feststellelement (15;64) durch Festziehen ermöglicht, dass der Schlitz (37) und damit auch die Bohrung (36) zusammengepresst wird.

9. Vorrichtung nach einem der Ansprüche 1 bis 8, **dadurch gekennzeichnet, dass** die am unteren Schwenkelement (6) an der Kontaktfläche mit dem mittleren Schwenkelement (5) angebrachte Verzahnung (12) kreisringförmig und konzentrisch zur Achse (38) ausgebildet ist.

10. Vorrichtung nach einem der Ansprüche 1 bis 9, **dadurch gekennzeichnet, dass** die am mittleren Schwenkelement (5) an der Kontaktfläche mit dem unteren Schwenkelement (6) angebrachte Verzahnung (11) kreisringförmig und konzentrisch zur Achse (38) ausgebildet ist.

11. Vorrichtung nach einem der Ansprüche 1 bis 10, **dadurch gekennzeichnet, dass** die am mittleren Schwenkelement (5) an der Kontaktfläche mit dem oberen Schwenkelement (4) angebrachte Verzahnung (10) als kreissegmentförmige Vertiefung (40) mit der senkrecht zur Achse (38) verlaufenden Drehachse (60) ausgebildet ist.

12. Vorrichtung nach einem der Ansprüche 1 bis 11, **dadurch gekennzeichnet, dass** die am oberen Schwenkelement (4) an der Kontaktfläche mit dem mittleren Schwenkelement (5) angebrachte Verzahnung (9) als halbkreisförmige Ausbuchtung (39) mit der senkrecht zur Achse (38) verlaufenden Drehachse (60) ausgebildet ist.

13. Vorrichtung nach einem der Ansprüche 1 bis 12, **dadurch gekennzeichnet, dass** die Knochenschrauben (3) zwischen der Backe (35) und dem oberen Schwenkelement (4) durch Spannschrauben (18) unabhängig von der Fixierung der Schwenkelemente (4;5;6) fixierbar sind.

14. Vorrichtung nach einem der Ansprüche 1 bis 13, **dadurch gekennzeichnet, dass** die am oberen Schwenkelement (4) angebrachte Spannfläche (47) auf ihrer Breite mit parallelen Vertiefungen (45) zur teilweisen Aufnahme der Knochenschrauben (3) versehen ist.

15. Vorrichtung nach einem der Ansprüche 1 bis 14, **dadurch gekennzeichnet, dass** die Backe (35) auf der der Spannfläche (47) am oberen Schwenkelement (4) zugewandten Seite auf ihrer Breite mit parallelen Vertiefungen (46) zur teilweisen Aufnahme der Knochenschrauben (3) versehen ist.

16. Vorrichtung nach einem der Ansprüche 1 bis 15, **dadurch gekennzeichnet, dass** ein als Feststellschraube ausgebildetes Feststellelement (64) auf beiden Stirnflächen mit je einem zur Aufnahme eines Werkzeuges zum Lösen und Anziehen der Feststellschraube dienenden Mittel (65;66) versehen ist.

## Claims

1. Apparatus (2) to externally affix parts of fractured bones and affixable to a longitudinal connecting element (1), comprising a jaw (35), an upper pivoting element (4), a middle pivoting element (5) and a lower pivoting element (6), the pivoting elements (4; 5; 6) being configured along at least one axis (38) in such manner that two pivoting elements (4; 5; 6) each are in contact, where
(a) at least two bone screws (3) or pins can be clamped between the jaw (35) and a clamping surface (47) configured at the upper pivoting element (4),
(b) the upper pivoting element (4) is rotatable relative to the middle pivoting element (5) about an axis of rotation (60) transversely to the axis (38) and clampable against the first end face (61) transverse to the axis (38) of the middle pivoting element (5),
(c) the lower pivoting element (6) is rotatable relative to the middle pivoting element (5) about the axis (38) and is clampable against the second end face (62) of this middle pivoting element (5),
(d) the lower pivoting element (6) is fitted with an end-to-end borehole (36) transverse to the axis (38) and allowing to shift the lower pivoting element (6) along the connecting element (1) and to rotate it about the central axis (7) of the borehole (36) relative to the connecting element (1), and
(e) the lower pivoting element (6) can be fixed in position on the connecting element (1),
**characterized in that**
(f) at least two of the pivoting elements (4; 5; 6) are fitted with serrations (9; 10; 11; 12) at the mutually touching contacting surfaces for the purpose of establishing positive connection of any two pivoting elements (4; 5; 6) in mutual contact and **in that** all three pivoting elements (4; 5; 6) are mutually affixable and the apparatus (2) is affixable to the connecting element (1) with a single affixation element (15;64).

2. Apparatus as claimed in claim 1, **characterized in that** the axis of rotation (60) is perpendicular to the axis (38).

3. Apparatus as claimed in either of claims 1 and 2, **characterized in that** the borehole (36) in the lower pivoting element (6) runs perpendicular to the axis (38).

4. Apparatus as claimed in one of claims 1 through 3, **characterized in that** the end faces (61; 62) of the middle pivoting element (5) are parallel.

5. Apparatus as claimed in one of claims 1 through 4, **characterized in that** the affixation element (15; 64) is a tightening screw.

6. Apparatus as claimed in claim 5, **characterized in that** the single affixation element (15) rests by the screw head (49) on a surface (52) at the lower pivoting element (6) and perpendicular to the axis (38) and **in that** this affixation element (15) can be screwed into a thread matching its own (50) and fitted on an arcuate insert (43) rotatably supported in the upper pivoting element (4).

7. Apparatus as claimed in claim 5 and 6, **characterized in that** the single affixation element (64) rests by the screw head (68) on a support surface (63) of an arcuate insert (43) rotatably resting in the upper pivoting element (4) and **in that** it can be threaded at the lower pivot element (6) into a thread matching its thread (67).

8. Apparatus as claimed in one of claims 1 through 5, **characterized in that** the pivoting element (6) is fitted on one side with a slit (37) running as far as the borehole (36) and **in that** an affixation element (15; 16) when tightened allows compressing the slit (37) and thereby also the borehole (36).

9. Apparatus as claimed in one of claims 1 through 8, **characterized in that** the serration (12) fitted to the lower pivoting element (6) on its contacting surface with the middle pivoting element (5) is circular and concentric to the axis (38).

10. Apparatus as claimed in one of claims 1 through 9, **characterized in that** the serration (11) fitted to the middle pivoting element (5) on its contacting surface with the lower pivoting element (6) is circular and concentric to the axis (38).

11. Apparatus as claimed in one of claims 1 through 10, **characterized in that** the serration (10) fitted to the middle pivoting element (5) on its contacting surface with the upper pivoting element (4) is an arcuate recess (40) of which the axis of rotation (60) runs perpendicular to the axis (38).

12. Apparatus as claimed in one of claims 1 through 11, **characterized in that** the serration (9) fitted at the upper pivoting element (4) on its contacting surface with the middle pivoting element (5) is a semi-circular bay (39) having an axis of rotation (60) perpendicular to the axis (38).

13. Apparatus as claimed in one of claims 1 through 12, **characterized in that** the bone screws (3) between the jaw (35) and the upper pivoting element (4) can be affixed by clamping screws (18) independently of the affixation of the pivoting elements (4; 5; 6).

14. Apparatus as claimed in one of claims 1 through 13, **characterized in that** the clamping surface (47) at the upper pivoting element (4) is fitted across its width with parallel recesses (45) partly seating the bone screws (3).

15. Apparatus as claimed in one of claims 1 through 14, **characterized in that** the jaw (35) is fitted at its side facing the clamping surface (47) of the upper pivoting element (4) with parallel recesses (46) across its width to partly seat the bone screws (3).

16. Apparatus as claimed in one of claims 1 through 16, **characterized in that** an affixation element (64) in the form of a tightening screw is fitted at both its end faces with a means (65; 66) to receive a tool to loosen or clamping the tightening screw.

## Revendications

1. Dispositif (2) de fixation externe de fragments d'os cassés, lequel peut être fixé sur un élément de liaison longitudinal (1) et comprend une mâchoire (35), un élément mobile supérieur (4), un élément mobile intermédiaire (5) et un élément mobile inférieur (6), et les éléments mobiles (4 ; 5 ; 6) sont disposés le long d'au moins un axe (38) de sorte que les éléments mobiles se touchent deux à deux (4 ; 5 ; 6), dans lequel
A) au moins deux vis à os (3) ou broches peuvent être serrées entre la mâchoire (35) et une surface de serrage (47) disposée sur l'élément mobile supérieur (4) ;
B) l'élément mobile supérieur (4) peut tourner, par rapport à l'élément mobile intermédiaire (5), autour d'un axe de rotation (60), lequel est transversal à l'axe (38), et peut être serré contre la première face frontale (61) de l'élément mobile intermédiaire (5) transversal à l'axe (38) ;
C) l'élément mobile inférieur (6) peut tourner, par rapport à l'élément mobile intermédiaire (5), autour de l'axe (38) et peut être serré contre sa deuxième face frontale (62) ;
D) l'élément mobile inférieur (6) est pourvu d'un trou traversant (36) s'étendant en travers à l'axe (38), lequel permet de déplacer l'élément mobile inférieur (6) sur l'élément de liaison (1) et de le faire tourner autour de l'axe central (7) du trou (36) par rapport à l'élément de liaison (1) ; et
E) l'élément mobile inférieur (6) peut être fixé sur l'élément de liaison (1);
**caractérisé en ce que**
F) au moins deux des éléments mobiles (4 ; 5 ; 6) sont pourvus, au niveau des surfaces de contact se touchant face à face, de dentures (9 ; 10 ; 11 ; 12) pour la liaison par emboîtement de deux éléments mobiles (4 ; 5 ; 6) en contact l'un avec l'autre et les trois éléments mobiles (4 ; 5 ; 6) peuvent être fixés les uns contre les autres et le dispositif (2) peut être fixé sur l'élément de liaison (1) à l'aide d'un seul élément de blocage (15 ; 64).

2. Dispositif selon la revendication 1, **caractérisé en ce que** l'axe de rotation (60) est perpendiculaire à l'axe (38).

3. Dispositif selon la revendication 1 ou 2, **caractérisé en ce que** le trou (36) dans l'élément mobile inférieur (6) est perpendiculaire à l'axe (38).

4. Dispositif selon l'une quelconque des revendications 1 à 3, **caractérisé en ce que** les faces frontales (61 ; 62) de l'élément mobile intermédiaire (5) sont parallèles.

5. Dispositif selon l'une quelconque des revendications 1 à 4, **caractérisé en ce que** l'élément de blocage (15 ; 64) est une vis de blocage.

6. Dispositif selon la revendication 5, **caractérisé en ce que** le seul élément de blocage (15) avec la tête de vis (49) prend appui sur une surface (52) perpendiculaire à l'axe (38) de l'élément mobile inférieur (6) et peut être vissé dans un taraudage, correspondant au filetage (50) de la vis de blocage, dans un élément d'insertion (43) en forme de segment de cercle disposé de manière à pouvoir tourner dans l'élément mobile supérieur (4).

7. Dispositif selon la revendication 5, **caractérisé en ce que** le seul élément de blocage (64) avec la tête de vis (68) prend appui sur une surface d'appui (63) d'un élément d'insertion (43) en forme de segment de cercle disposé de manière à pouvoir tourner dans l'élément mobile supérieur (4) et peut être vissé dans un taraudage, correspondant au filetage (67) de la vis de blocage, dans l'élément mobile inférieur (6).

8. Dispositif selon l'une quelconque des revendications 1 à 5, **caractérisé en ce que** l'élément mobile (6) est pourvu, d'un côté, d'une fente (37) s'étendant jusqu'au trou (36), et un élément de blocage (15 ; 64) permet, par serrage, de compresser à la fois la fente (37) et le trou (36).

9. Dispositif selon l'une quelconque des revendications 1 à 8, **caractérisé en ce que** la denture (12) disposée sur l'élément mobile inférieur (6) au niveau de la surface de contact avec l'élément mobile intermédiaire (5) est conçue en forme de cercle et de manière concentrique à l'axe (38).

10. Dispositif selon l'une quelconque des revendications 1 à 9, **caractérisé en ce que** la denture (11) disposée sur l'élément mobile intermédiaire (5) au niveau de la surface de contact avec l'élément mobile inférieur (6) est conçue en forme de cercle et de manière concentrique à l'axe (38).

11. Dispositif selon l'une quelconque des revendications 1 à 10, **caractérisé en ce que** la denture (10) disposée sur l'élément mobile intermédiaire (5) au niveau de la surface de contact avec l'élément mobile supérieur (4) est conçue en forme d'évidement circulaire (40) avec l'axe de rotation (60) perpendiculaire à l'axe (38).

12. Dispositif selon l'une quelconque des revendications 1 à 11, **caractérisé en ce que** la denture (9) disposée sur l'élément mobile supérieur (4) au niveau de la surface de contact avec l'élément mobile intermédiaire (5) est conçue en forme de renflement hémisphérique (39) avec l'axe de rotation (60) perpendiculaire à l'axe (38).

13. Dispositif selon l'une quelconque des revendications 1 à 12, **caractérisé en ce que** les vis à os (3) peuvent être fixées entre la mâchoire (35) et l'élément mobile supérieur (4) à l'aide de vis de serrage (18) indépendamment de la fixation des éléments mobiles (4 ; 5 ; 6).

14. Dispositif selon l'une quelconque des revendications 1 à 13, **caractérisé en ce que** la surface de serrage (47) disposée sur l'élément mobile supérieur (4) est pourvue, sur sa largeur, de rainures parallèles (45) pour loger en partie les vis à os (3).

15. Dispositif selon l'une quelconque des revendications 1 à 14, **caractérisé en ce que** la mâchoire (35), sur la face tournée vers la surface de serrage (47) sur l'élément mobile supérieur (4), est pourvue, sur sa largeur, de rainures parallèles (46) pour loger en partie les vis à os (3).

16. Dispositif selon l'une quelconque des revendications 1 à 15, **caractérisé en ce qu'**un élément de blocage (64) conçu sous forme de vis de blocage est pourvu sur les deux faces frontales d'un moyen (65 ; 66) servant à recevoir un outil pour desserrer et serrer la vis de blocage.
